# EUROPEAN PATENT APPLICATION

(11) **EP 2 540 814 A1**
(43) Date of publication of application: **02.01.2013**
(21) Application number: 10831187.9
(22) Date of filing: 18.11.2010
(51) Int. Cl.: C12M 1/00

(54) **PHOTOBIOREACTOR FOR THE CONTINUOUS CULTURE OF MICROALGAE AND A MODULAR SYSTEM COMPRISING SAID PHOTOBIOREACTORS**

(30) Priority: 20.11.2009 ES 200931036
(71) Applicant: Consejo Superior De Investigaciones Científicas (CSIC), 28006 Madrid (ES)
(72) Inventor: ASENSIO BELTRAN, Enrique, E-12595 Cabanes (Castellón la Plana) (ES)
(74) Representative: Pons Ariño, Angel
(86) International application number: PCT/ES2010/070749
(87) International publication number: WO 2011/061380

(57) **Abstract**

The invention relates to a photobioreactor for the continuous culture of microalgae and to a modular system comprising said photobioreactors. The system includes photobioreactors (22) controlled by programmers (16) and interconnected to form modules controlled by a control unit. Each photobioreactor (22) includes at least one transparent culture chamber (1) allowing the passage of an aeration circuit (2), a culture medium circuit (3) and, optionally, a heat-exchange circuit. The aeration circuit (2) supplies the culture chamber (1) with CO2 and removes 02 therefrom. The culture medium circuit (3) introduces fertiliser and water and extracts the culture, said circuit (3) comprising a first container for water, a second container (4) for the culture medium, means (7) for sterilising the culture medium, and a first pump (6) for conveying the culture medium towards the chamber (1). Ash from pruning or brine from desalination plants can be used as the culture medium

## Description

### OBJECT OF THE INVENTION

The present invention may be included in the field of aquaculture, particularly in the field of microalgae cultivation.

The object of the invention is centred on a photobioreactor for the continuous cultivation of microalgae, as well as a modular system that comprises said photobioreactors.

### BACKGROUND OF THE INVENTION

In the last few years, advances have been made in the conceptual design of photobioreactors for microalgae cultivation, it is generally considered that in order to attain massive microalgae cultivation, it is essential to adequately choose the design of the reactor that will be employed. To that end, a number of parameters must be taken into account depending on the microorganism that is going to be used, as well as optimal growing conditions and resistance to environmental variations, and also economic parameters such as the value of the product obtained, necessary investment capital, estimated operation costs, etc.

The work areas in this field imply:
- Fluid-dynamic characterization and energy and matter transfers.
- Quantification of the stress phenomena to which the cells are subjected.
- Determination of light availability by individual cells.
- Determination and control of the operational variables (pH, temperature, CO2, 02, etc.).
- Productivity of the system.

These areas are interrelated, thus, the fluid-dynamic characterization and energy and matter transfer have an influence and will be, at the same time, determined by the stress produced on the cells or their needs with regards to temperature, as well as on the operational variable to be controlled, such as oxygen removal, CO₂ and pH control, etc. All of these have an influence on the productivity of the system. Generally, it is possible to establish different mixing conditions in the same photobioreactor in order to manipulate the rate of illumination and thus the rate of photosynthesis. The systems based in the aeration of the culture with compressed air (airlift), are commonly used due to their simplicity and because they may be designed to produce a small cutting effort that will not cause mechanical damage on the cells. [4]

The agitation speed must be enough to maintain a turbulence rate which, on one hand, prevents growth on the reactor's wall or the sedimentation of the cells and, on the other, ensures favourable illumination of the cells through an appropriate frequency of the light/shadow cycles to achieve an intense photosynthesis. [3]

To achieve cultivations with an ultra-high cellular density, a variety of geometries of photobioreactors and illumination sources have been used, such as flat vertical reactors with white light fluorescent lamps, reactors quasi-internally illuminated with diodes, reactors internally illuminated with fibre optic, and inclined flat reactors with solar illumination kept outside.

As an example, in the case of the Dunaliella salina algae, these are very well adapted to propagate in environments ranging from less salt concentration than ocean saltwater (0,1 NaCl M) to saturated solutions. (> NaCl 5M).

Up to this moment, four mediums for large-scale production of Dunaliella have been developed. In the first one, known as extensive cultivation, no agitation is used and the environmental control is minimal. In order to decrease predator attacks (ciliates, amoebas, artemia, etc.), very high salt concentrations are used. In these conditions, algae grow very slowly. The productivity of this type of cultivation is low and thus large areas are needed for their commercial exploitation; however, operational costs are very low. In the second medium, known as intensive cultivation, there is an attempt to control all factors of cellular growth. The tanks are normally rectangular, and are aligned to form canals with a variable size. In this type of cultivation, about 200 mg of B-carotene/m² per day are obtained as an average throughout the year, which is equivalent to 2 Kg/Ha per day. Between these two systems there is a third one developed in Australia and China, where the length has been increased by a factor of 10, there is no agitation but there is a partial control. The fourth system is highly intensive and is developed in enclosed bioreactors. This system has earned special attention since the last decade, although it is still in an experimental stage. [2]

In another interesting example, for the cultivation of Spirulina sp, the production systems vary between systems with more or less technified tanks, where, depending on the problems associated with natural water supply and the microalgae's potential as a source of biomass and biomolecules, circular tanks are used, raceways (elongated tanks) or cascading tanks. Circular tanks are still used in Japan, Taiwan and Indonesia.

The tanks with a raceway shape (in a row), are used in Israel, the United States of America, China and other countries. Fertilizer is used and the culture is stirred with a paddle wheel. The cellular concentration may be maintained at around 0.5 g/l and a productivity of around 25 g/m². per day has been widely reported. On the other hand, in order to obtain high cellular concentrations and a higher level of control over the culture, several forms of photobioreactors have been proposed. [1] These include straight horizontal tubes connected with U-shaped curves, photobioreactors of the α type with crossed tubes placed in an angle with the horizontal (and) flexible tubes rolled around a vertical cylinder.

Another type is the flat bioreactors, usually raised in an angle with the horizontal and in some cases in vertical with the ground. [5]

Different culture mediums and various temperatures have been tested, in all cases the medium is alkaline and generally sodium bicarbonate is found in higher proportions.

The system is completed with the harvesting of the product which typically comprises careful operations of concentration, filtration, centrifugation, pasteurization and careful drying though spray dryer, in order to prevent the deterioration of some nutrients.

From what was previously described, it is implied that production through enclosed and axenic systems similar to those used in a laboratory requires very high investment and maintenance costs, only accessible to large corporations while, on the other hand, production through low-cost open systems does not guarantee the performance or quality of the product.

Therefore, the technical problem that is raised, is about the need for a microalgae production with a cost/product relation optimized through the design of photobioreactors that allow reaching a high average productivity without straining the culture, with moderate costs and with a high degree of automatization and control, so that it enables the optimization of the growth curve in conditions, which while not entirely axenic, are at least bounded and reliable, besides scalability.

### REFERENCES INDEX:

[1] Acien Fernandez, F.G., Fernandez Sevilla, J.M. , Sanchez Perez, J. A., Molina Grima, E., Chisti, Y., Airlift-driven external-loop tubular photobioreactors for outdoor production of microalgae: assessment of design and performance. Chemical engineering Science 56 (2001) 2721 - 2732.
[2] Garcia Gonzalez M., Manzano J.C., Moreno J., Guerrero M.G. Biotecnología del cultivo de Dunaliella salina en el litoral andaluz. Consejeria de Agricultura y Pesca. Junta de Andalucia. Sevilla 2000.
[3] Molina E., Acien F.G., Garcia F., Chisti Y. Photobioreactors: Light regime, mass transfer, and scaleup. J. Biotechnol. 70: 231 - 248 (1999)
[4] Richmond, A., Boussiba, S., Vonshak, A. & Kopel, R., A new tubular reactor for mass production of microalgae outdoors. Journal of Applied Phycology 5: 327 - 332, (1993).
[5] Richmond A., Cheng-Wu Zhang. Optimization of a flat plate glass reactor for mass production of Nannochloropsis sp. outdoors. Journal of Biotechnology (2001).

### DESCRIPTION OF THE INVENTION

The present invention solves the technical problem raised with the use of a modular photobioreactor for the continuous cultivation of microalgae whose operation is based on the conjunction of a bubbling column and a localized watering system. On one hand, the bubbling column is similar in the present invention to a receptor plant of a culture medium (watering and fertilizing) of a localized watering system and, on the other hand, by means of the invention, a dripping system for watering is adapted to an aquaculture culture, being obtained in a watering system through controlled flooding inside a culture chamber, since the microalgae object of cultivation lives submerged. A subsequent drainage is identified with the harvesting or recollection.

The invention solves the technical problem of obtaining microalgae cultures in a photobioreactor with moderately high production and high scalability without the need of high investments, due to its easy implementation, its low cost and versatility as well as its capacity to reuse alternative raw materials, at times obtained by the operation itself, such as the pruning ashes or the desalination brines. In addition, the use of the invention's photobioreactor allows for high profitability which enables the attainment of an optimized product/cost relation since said bioreactor does not exert high cutting stress over the culture's cells, requires low maintenance and features a high degree of automatization, further enabling a cultivation density (litres/m²) equal or higher than the tanks system with an area of illumination three times as large.

The description that follows is applied preferably to enclosed systems, since they allow for a better control over the operational conditions. Even more preferred among the enclosed systems, are those of the photobioreactor type with a bubbling column shape, continuous mixture and the modular type, which enables scaling through modular grouping of photobioreactors.

As was mentioned above, the invention relates to a modular system of enclosed photobioreactors for the cultivation of microalgae in a continuous culture medium. The bioreactors are connected to each other in a series or parallel to each other according to modules that, at the same time, may be grouped into sectors, etc., so that each higher grouping unit from a simple photobioreactor is controlled by a programmer.

Each photobioreactor is constituted by one or several transparent tubes or cells, known as culture chambers, placed in a vertical position, which preferably comprise open ends (upper and lower) which are adapted to be covered by upper and lower closing means, respectively, located on said ends and to allow for the installation of an aeration system and a culture medium circuit inside of the culture chamber. Optionally, the analogous installation of a heat exchanger is also contemplated. Preferably, a light support with a trunk-pyramid structure and triangular base is used for the vertical placement of the tubes. This way, the three vertices of the triangular base are admitted on a single plane, which leads to the stability of the support. In addition, the placement according to said light support facilitates the transportability of the bioreactor.

The culture medium circuit allows the entry into the culture chamber of the culture medium and the exit of the culture. The culture medium is preferably constituted by pruning ashes or brines coming from desalination, as examples of alternative culture mediums that are environmentally beneficial.

The aeration circuit allows for supply of the CO₂ inside the culture chamber and for removal of the O₂ from said culture chamber at the same time. Said aeration circuit comprises a compressor and a filter, through which the exterior air is pushed towards the bottom of the culture chamber and subsequently towards the outside by way of bubbles, with a flow that is adjustable in intensity and bubble size through the compressor and some diffusers. The regulation of the aeration circuit is independent from the control established by the programmer.

The heat exchange circuit, which is optional, may be composed of a heat exchanger located inside the culture chamber and, in certain location, or non-with a non-exclusive nature, it may be alternatively constituted by a plastic cover made of a transparent material, for the cold periods, or by means of a humidifying means during warm periods.

The culture medium circuit comprises a source or first water deposit, a second deposit where the culture medium is prepared, some means of sterilization of the culture medium and a first pump that is adapted to cause the circulation of the water and/or the culture medium towards the culture chamber from the first deposit and the second deposit respectively. The culture medium passes through the first pump regulated by the programmer towards the culture chamber, where it exits either through some overflows or, alternatively, through a second pump, from which it reaches some filtration means, where a small part is separated as harvested product and the other part goes through recycling means where it is again divided, with some part of it going to the culture chamber and the other going to a detritus deposit.

### DESCRIPTION OF THE DRAWINGS

To complement the description being made and in order to help better understand the features of the invention, according to a preferred practical embodiment thereof, a set of drawings is attached as an integral part of said description, wherein the following is shown as way of illustration but not limited to:

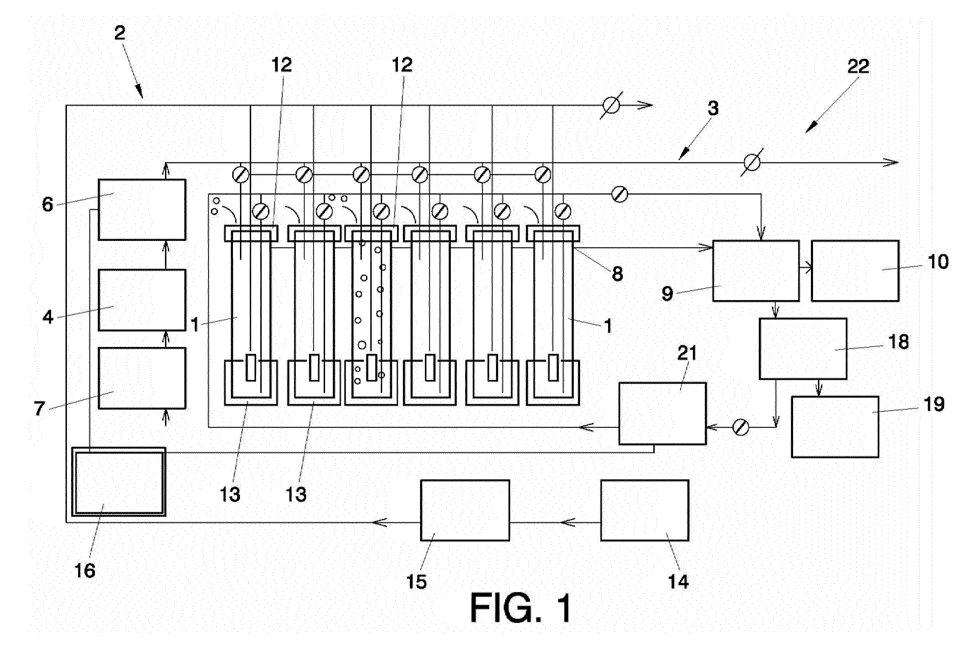

Figure 1. - Shows a diagram of the structure and operation of the photobioreactor of the invention.

### PREFERRED EMBODIMENT OF THE INVENTION

The modular system of the invention is formed by individual photobioreactors (22) interconnected in a series or in parallel and grouped into modules, which, at the same time, may be grouped into sectors, etc. The operation of each photobioreactor (22) is controlled by a programmer (16), while the operation of each higher grouping (module, sector, etc.) from simple photobioreactors (22) is directed by two control units (not represented).

The photobioreactor (22) shown in figure 1 comprises a culture chamber (1) with a tube shape and made of methacrylate, covered on both ends by closing means both upper (12) and lower (13) ends. The culture chambers (1) are placed in a vertical position and the upper closing means (12) are adapted, through perforations (not represented), to house the input of an aeration circuit (2) and of a culture medium circuit (3). The diameter of the perforations and of the chambers (1) is determined according to the hydraulic theory and the localized watering knowlend.

The aeration circuit (2) enables the supply of CO₂ to the inside of the culture chamber (1) and to remove at the same time the O₂ from said culture chamber (1). Said aeration circuit (2) comprises a compressor (14) and a filter (15), through which the exterior air is pushed towards the bottom of the culture chamber (1) and subsequently towards the outside by way of bubbles, with a flow that is adjustable in intensity and bubble size, through a compressor (14) and some diffusers (not represented).

The culture medium circuit (3) comprises an first water deposit (not represented), a second deposit (4) for the culture medium, some sterilization means (7) for the culture medium and an first pump (6) adapted to cause the circulation of the culture medium and/or the water towards the culture chamber (1) from the first deposit (4) and the second deposit, respectively. The culture medium passes through the first pump (6), regulated by the programmer (16), towards the culture chamber (1), where it exits, either through overflows (8) or alternatively through a second pump (not shown), from which it reaches to some filtration means (9), where a small part is separated a harvested product, through some harvesting means (10) which comprise some draining means, while the other part goes to some recycling means (18), adapted to separate detritus from the filtering and conduct said filtering without detritus through a third pump (21) towards the exterior of the culture chamber (1). The detritus is conducted to a detritus deposit (19), where a part that is capable of being transported towards the second deposit (4) of the culture medium, is recovered.

The culture medium circuit (3) allows the entry into the culture chamber (1) of the culture medium (fertilizer and water) and the exit of the culture. The culture medium is constituted by pruning ashes or brines coming from desalinations, which constitute alternative culture mediums that are environmentally beneficial.

## Claims

1. Photobioreactor (22) for the continuous cultivation of microalgae, which comprises at least a culture chamber (1), made of a transparent material and adapted to be held in a substantially vertical position, containing a microalgae culture,
**characterized in that** it additionally comprises:
- an aeration circuit (2) adapted to provide the culture contained in the culture chamber (1) with a supply of CO₂ through a compressor (14) and a filter (15) and remove O₂, and
- a culture medium circuit (3) adapted to perform entry and exit of a culture medium and water in the culture chamber (1), through a dripping system.

2. Photobioreactor (22) for the continuous cultivation of microalgae, according to claim 1, **characterized in that** the culture medium circuit (3) comprises:
- a first water deposit,
- a second deposit (4) for the culture medium,
- sterilization means (7) for the culture medium,
- a first pump (6) adapted to cause the circulation of water and/or the culture medium towards the culture chamber (1) from the first deposit and the second deposit (4) respectively.

3. Photobioreactor (22) for the continuous cultivation of microalgae according to any of claims 1 or 2, **characterized in that** it additionally incorporates a heat exchange means with the culture.

4. Photobioreactor (22) for the continuous cultivation of microalgae according to claim 3, **characterized in that** the heat exchange means is selected from:
- a heat exchanger installed inside the culture chamber (1),
- a cover made of a transparent material, for cold periods, and
- humidifying means, for warm periods.

5. Photobioreactor (22) for the continuous cultivation of microalgae according to claim 1, **characterized in that** the culture chamber (1) is open on at least one of its ends, incorporating some upper (12) and lower (13) closing means placed on said ends, with said closing means being adapted to allow passage to the aeration circuit (2), to the culture medium circuit (3) and to the heat exchange means.

6. Photobioreactor (22) for the continuous cultivation of microalgae according to claim 1, **characterized in that** the culture medium is selected from at least one the elements from the following list:
- pruning ashes,
- brines from desalination.

7. Photobioreactor (22) for the continuous cultivation of microalgae according to claim 1 **characterized in that** it additionally incorporates a recollection line which comprises:
- an overflow (8) or a second pump which are adapted to allow the exit of the culture from the culture chamber (1),
- some filtration means (9) for the culture, adapted to produce filtered culture, and
- some harvesting means (10) for the culture, which comprise some means for draining the culture.

8. Photobioreactor (22) for the continuous cultivation of microalgae according to any of claims 1 and 7, **characterized in that** it additionally comprises some recycling means (18) adapted to separate the detritus from the filtered culture and to conduct the filtered culture without detritus through a third pump (21) towards the interior of the culture chamber (1) or towards the overflow (8) or second pump.

9. Photobioreactor (22) for the continuous cultivation of microalgae according to claim 1 **characterized in that** it comprises a pyramidal frusta support with a triangular base adapted to hold the culture chamber (1) in a vertical position and to transport the photobioreactor.

10. Modular system which comprises photobioreactors (22) described in any of the previous claims, the photobioreactors (22) being interconnected and grouped into modules, **characterized in that** it additionally comprises a control unit adapted to control the operation of the modules.
